# EUROPEAN PATENT APPLICATION

(11) **EP 4 467 173 A1**
(43) Date of publication of application: **27.11.2024**
(21) Application number: 24176625.2
(22) Date of filing: 17.05.2024
(51) Int. Cl.: A61M 5/142

(54) **AMBULATORY INFUSION PUMP DEVICE WITH AN EPICYCLIC INSERTER**

(30) Priority: 22.05.2023 US 202363503543 P; 29.04.2024 US 202418648855
(71) Applicant: Medtronic MiniMed, Inc., Northridge, CA 91325 (US)
(72) Inventor: Smith, Roger E., Northridge, 91325 (US)
(74) Representative: Zimmermann & Partner Patentanwälte mbB

(57) **Abstract**

In some embodiments, an infusion device (100) can include a reservoir (408) configured to hold a medicament, a first epicyclic gear arrangement (601a) comprising a first output, a second epicyclic gear arrangement (601b) comprising a second output, a first trocar (674) coupled to the first output and movably coupled to a sensor electrode(670), and a second trocar (684) coupled to the second output and movably coupled to an infusion cannula (680) that is fluidically coupled to the reservoir. Actuation of the first epicyclic gear arrangement can drive linear travel of the first trocar and/or the sensor electrode, and actuation of the second epicyclic gear arrangement can drive linear travel of the second trocar and/or the infusion cannula.

## Description

### CROSS REFERENCE TO RELATED APPLICATIONS

This application claims the benefit of U.S Provisional Application Ser. No. 63/503,543, filed May 22, 2023, and entitled "Ambulatory Infusion Pump Device with an Epicyclic Inserter," which is incorporated herein by reference in its entirety.

### TECHNICAL FIELD

The present technology relates generally to medical devices, such as insulin delivery devices with integrated continuous glucose monitors.

### BACKGROUND

Ambulatory infusion pumps are relatively small, at least substantially self-contained devices that are used to introduce drugs and other infusible substances (collectively "medicament") into users' bodies. Some infusion pumps are configured to be worn on a belt, carried in a clothing pocket, or the like. Other infusion pumps are configured to be adhered to skin in a patch-like fashion. Infusion pumps are advantageous in that they may be used to, for example, subcutaneously introduce (or "infuse") medicament on an ongoing or even continuous basis outside of a clinical environment. Infusion pumps are also advantageous in that they greatly reduce the frequency of subcutaneous access events such as needle-based shots. One example of a medicament that may be introduced by an infusion pump is a liquid formulation of insulin. Other exemplary medicaments that may be introduced by an infusion pump include, but are not limited to, drugs that treat cancers and drugs that suppress the perception of pain.

In some instances, ambulatory infusion pumps may automatically dispense medicament (e.g., insulin) based on measurements obtained by a real-time monitoring device. In the case of an insulin infusion pump, such a real-time monitoring device can take the form of a continuous glucose monitor (CGM). Examples of such CGMs include wearable devices having a sensor component that can be inserted into the user's skin to record a user's glucose levels continuously or periodically over time.

The use of an infusion pump device and a separate sensor device creates complexity for the user and requires significant space on a user's skin for implementation. Although combination devices have been proposed that integrate both an infusion pump and a CGM, such devices are generally bulky and expensive. Additionally, in combination devices, the sensor component may be positioned too near to the insulin delivery cannula to obtain accurate physiological insulin readings of the user. Accordingly, there remains a need to develop improved ambulatory infusion pump devices with integrated sensors.

### SUMMARY

Generally, in some embodiments in accordance with the present technology, an infusion device can include a reservoir configured to hold a medicament, a first epicyclic gear arrangement comprising a first output, a second epicyclic gear arrangement comprising a second output, a first trocar coupled to the first output and movably coupled to a sensor electrode, and a second trocar coupled to the second output and movably coupled to an infusion cannula, wherein the infusion cannula is fluidically coupled to the reservoir. Actuation of the first epicyclic gear arrangement can drive linear travel of the first trocar and the sensor electrode, and actuation of the second epicyclic gear arrangement can drive linear travel of the second trocar and the infusion cannula.

In some embodiments, the first epicyclic gear arrangement can include a first ring gear and a first planetary gear engaged with the first ring gear, and the second epicyclic gear arrangement can include a second ring gear and a second planetary gear engaged with the second ring gear. At least one shaft can be coupled to one or both of the first and second epicyclic gear arrangements. For example, in some embodiments, such a shaft or shafts can include a first rotatable hub coupled to the first planetary gear and a second rotatable hub coupled to the second planetary gear. In some embodiments, the first and second rotatable hubs can be coupled to each other.

In some embodiments, the first output of the first epicyclic gear arrangement can be the first planetary gear, and the second output of the second epicyclic gear arrangement can be the second planetary gear. The first trocar can be pivotably coupled to the first output and constrained to move along a first linear path, and the second trocar can be pivotably coupled to the second output and constrained to move along a second linear path. In some embodiments, linear travel of the first trocar and the sensor electrode can be laterally spaced apart from linear travel of the second trocar and the infusion cannula. In some embodiments, the sensor electrode can be movably received within a recess of the first trocar, and/or the second trocar can be movably received in (e.g., extend within) a lumen of the infusion cannula.

The infusion device can further include an actuator coupled to the first and second epicyclic gear arrangements. For example, in some embodiments the actuator can include a torsion spring. The torsion spring can be configured such that a first range of rotation of the torsion spring is configured to drive the first trocar, the sensor electrode, the second trocar, and the infusion cannula in a first direction. Additionally or alternatively, the torsion spring can be configured such that a second range of rotation of the torsion spring is configured to retract the first trocar and the second trocar in a second direction opposite the first direction. In some embodiments, the infusion device can include a trigger configured to activate the actuator. The trigger can, for example, include a manual trigger mechanism (e.g., button) or a remote trigger mechanism.

In some embodiments, the infusion device can include a housing configured to at least partially surround the reservoir, the first epicyclic gear arrangement, and/or the second epicyclic gear arrangement. The first trocar, the sensor electrode, the second trocar, and the infusion cannula can be configured to linearly travel to extend at least partially outside the housing. In some embodiments, the infusion device can include a sensor carrier slidingly engaged with the housing and coupled to the sensor electrode, and/or a cannula carrier slidingly engaged with the housing and coupled to the infusion cannula. In some embodiments, at least one latch can be configured to selectively limit the position of one or both of the sensor carrier and the cannula carrier relative to the housing.

Generally, in some embodiments in accordance with the present technology, a device can include a first epicyclic gear arrangement including a first ring gear and a first planet gear engaged with the first ring gear, a second epicyclic gear arrangement including a second ring gear and a second planet gear engaged with the second ring gear, a first member (e.g., piercing member) coupled to the first planet gear, and a second member (e.g., piercing member) coupled to the second planet gear. Actuation of the first and second epicyclic gear arrangements can drive travel of the first and second members, respectively. In some embodiments, the first member can include a first trocar and/or the second member can include a second trocar. One or both trocars can be movably coupled to a respective medical device. The first trocar can, for example, be movably coupled to a sensor (e.g., sensor electrode) and/or the second trocar can be movably coupled to a cannula (e.g., infusion cannula).

In some embodiments, the device can further include at least one shaft coupled to one or both of the first and second epicyclic gear arrangements. For example, the shaft or shafts can include a first rotatable hub coupled to the first planetary gear, and a second rotatable hub coupled to the second planetary gear. In some embodiments, the first and second hubs are coupled together (e.g., splined together).

In some embodiments, actuation of the first epicyclic gear arrangement can drive the first member in a first linear path and actuation of the second epicyclic gear arrangement can drive the second member in a second linear path spaced apart (e.g., laterally) from the first linear path.

Generally, in some embodiments in accordance with the present technology, a method for operating an infusion device arranged over skin of a user can be used for delivering medicament to a user. The infusion device can include at least one epicyclic gear arrangement, a sensor electrode, and an infusion cannula fluidically coupled to a reservoir holding a medicament. The method can include actuating the at least one epicyclic gear arrangement, thereby driving at least one of the sensor electrode and the infusion cannula into the skin of the user. The method can further include obtaining one or more physiological measurements of the user via the sensor electrode, and delivering medicament to the user via the infusion cannula based at least in part on the one or more physiological measurements. In some embodiments, the one or more physiological measurements can include an analyte measurement (e.g., blood glucose measurement), and/or the medicament can include insulin.

Actuating the epicyclic gear arrangement(s) can, for example, include driving the sensor electrode in a first linear path and the infusion cannula in a second linear path spaced apart from the first linear path. In some embodiments, the method includes receiving a trigger command to actuate the epicyclic gear arrangement(s). The trigger command can, for example, include a manual trigger mechanism (e.g., button) or a remote trigger mechanism.

In some embodiments, the infusion device can include a first trocar movably coupled to the sensor electrode and a second trocar movably coupled to the infusion cannula. In these embodiments, actuating the at least one epicyclic gear arrangement a first amount can drive the first and second trocars into the skin of the user, and actuating the at least one epicyclic gear arrangement a second amount can retract the first and second trocars from the skin of the user while leaving the sensor electrode and the infusion cannula in the skin of the user.

Additionally or alternatively, in some embodiments, the epicyclic gear arrangement can include a first epicyclic gear arrangement associated with the sensor electrode and a second epicyclic gear arrangement associated with the infusion cannula. The infusion device can, in some embodiments, include at least one shaft coupled to one or both of the first and second epicyclic gear arrangements. The infusion device can include an actuator configured to drive or otherwise rotate the shaft or shafts. For example, the actuator can include a torsion spring.

Generally, in some embodiments in accordance with the present technology, a device can include an insertion assembly including a first insertion means movably coupled to a sensor electrode, a second insertion means movably coupled to an infusion cannula, and a drive means coupled to the first and second insertion means so as to advance the sensor electrode and the infusion cannula. In some embodiments, the infusion cannula can be fluidically coupled to a reservoir. Additionally or alternatively, the first insertion means can include a first trocar and/or the second insertion means can include a second trocar.

In some embodiments, the drive means can be configured to advance the first insertion means and the sensor electrode along a first linear path, and advance the second insertion means and the infusion cannula along a second linear path spaced apart from the first linear path (e.g., laterally spaced apart). The drive means can be further configured to retract the first insertion means along the first linear path and retract the second insertion means along the second linear path (e.g., independent of the sensor electrode and the infusion cannula). In some embodiments, the drive means includes at least one epicyclic gear arrangement. For example, the epicyclic gear arrangement(s) can include a first epicyclic gear arrangement associated with the sensor electrode and a second epicyclic gear arrangement associated with the infusion cannula. In some embodiments, the drive means can include an actuator (e.g., torsion spring) coupled to one or more epicyclic gear arrangements.

Generally, in some embodiments in accordance with the present technology, a device can include an epicyclic gear arrangement comprising a ring gear and a planet gear engaged with the ring gear, a trocar pivotably coupled to the planet gear and constrained to travel in a linear path, and an instrument movably coupled to the trocar, where actuation of the epicyclic gear arrangement drives travel of the trocar and the instrument along the linear path. The instrument can be aligned with a longitudinal axis of the trocar (e.g., coaxially aligned). For example, the instrument can be arranged within a longitudinal recess or a lumen of the trocar. Alternatively, the trocar can be arranged within a longitudinal recess or a lumen of the instrument. In some embodiments, the instrument can be a sensor electrode or an infusion cannula.

In some embodiments, the device can include an actuator such as a torsion spring coupled to the epicyclic gear arrangement. In some embodiments in which the actuator includes a torsion spring, a first range of rotation of the torsion spring can be configured to drive the trocar and the instrument in a first direction, and a second range of rotation can be configured to retract the trocar in a second direction opposite the first direction.

Additionally or alternatively, in some embodiments the device can include a mount (e.g., a feature or component of a housing in the device) and a carrier slidingly engaged with the mount, where the instrument can be coupled to the carrier. In some embodiments, the device can further include one or more latches configured to selectively limit a position of the carrier relative to the mount. For example, the device can include a one-way latch (e.g., ratchet) that helps retain the carrier in or near a particular position relative to the mount, which can fix the position of the instrument relative to the mount. In some embodiments, the latch or latches can help hold the instrument at a particular axial position relative to the housing (e.g., fully extended) while enabling the trocar to be disengaged from the instrument and movable along the linear path without the instrument.

Furthermore, in some embodiments the device can include a second epicyclic gear arrangement including a second ring gear and a second planet gear engaged with the second ring gear. The device can further include a second trocar pivotably coupled to the second planet gear and constrained to travel in a second linear path, and a sensor electrode movably coupled to the second trocar. Actuation of the second epicyclic gear arrangement can drive travel of the second trocar and the sensor electrode along the second linear path.

Further described herein is that in some embodiments, an infusion device can include a reservoir configured to hold a medicament, a first epicyclic gear arrangement comprising a first output, a second epicyclic gear arrangement comprising a second output, a first trocar coupled to the first output and movably coupled to a sensor electrode, and a second trocar coupled to the second output and movably coupled to an infusion cannula that is fluidically coupled to the reservoir. Actuation of the first epicyclic gear arrangement can drive linear travel of the first trocar and/or the sensor electrode, and actuation of the second epicyclic gear arrangement can drive linear travel of the second trocar and/or the infusion cannula.

Other technical features may be readily apparent to one skilled in the art from the following figures, descriptions, and claims.

### BRIEF DESCRIPTION OF THE DRAWINGS

Many aspects of the present disclosure can be better understood with reference to the following drawings. The components in the drawings are not necessarily to scale. Instead, emphasis is placed on clearly illustrating the principles of the present disclosure.
FIG. 1 is a perspective view of an example infusion device with an integrated sensor that includes a disposable assembly and a durable assembly.
FIG. 2 is a perspective view of the device shown in FIG. 1 with the durable assembly removed.
FIG. 3 is a perspective view of the disposable assembly of the device shown in FIG. 2, with a portion of a device housing omitted for clarity.
FIGS. 4A and 4B are assembled and exploded views, respectively, of an example epicyclic inserter arrangement.
FIGS. 5A-5F are illustrative schematics of various stages of operation of the example epicyclic inserter arrangement shown in FIGS. 4A and 4B.
FIG. 5G is an illustrative schematic of gear relationships in an example epicyclic inserter arrangement.
FIGS. 6A and 6B are assembled and partially exploded perspective views, respectively, of an example epicyclic insertion assembly.
FIGS. 7A and 7B are assembled and partially exploded perspective views, respectively, of the example epicyclic insertion assembly shown in FIGS. 6A and 6B.
FIG. 8 is a perspective cross-sectional view of an example trigger mechanism in an epicyclic insertion assembly.
FIG. 9 is a perspective cross-sectional view of the example epicyclic insertion assembly shown in FIGS. 6A and 6B.
FIGS. 10A and 10B are front and rear perspective views, respectively, of an example epicycle insertion assembly in an unfired stage of operation.
FIGS. 11A and 11B are front and rear perspective views, respectively, of an example epicyclic insertion assembly in a partially fired stage of operation.
FIGS. 12A and 12B are front and rear perspective views, respectively, of an example epicyclic insertion assembly in a partially fired stage of operation.
FIGS. 13A and 13B are front and rear perspective views, respectively, of an example epicyclic insertion assembly in a fired stage of operation.
FIG. 14 is a side view of an example carrier latch mechanism in an epicyclic insertion assembly.
FIG. 15 is a schematic of a method of operation of an infusion device.

### DETAILED DESCRIPTION

### I. Overview

The present technology relates to wearable devices that can both deliver medicament (e.g., via infusion using a cannula inserted into a user's skin) and monitor one or more physiological parameters (e.g., via a sensor electrode or other sensing element inserted into the user's skin). In some embodiments, the medicament delivered can include insulin. In some embodiments, the sensing electrode can be configured to obtain one or more analyte measurements, such as glucose measurements that can be used to derive or infer the user's blood glucose levels. According to some examples, the device can include a housing that at least partially contains the components therein, including a reservoir, one or more actuators configured to deliver medicament, and an insertion assembly to drive an infusion cannula and sensor electrode out of the housing and into the user's skin. In operation, the housing can be placed over and adhered to the user's skin. After applying the device to the skin, the insertion assembly can be activated (e.g., by the user, automatically by the device, etc.) to insert the infusion cannula and the sensor electrode subcutaneously. While it is generally desirable to minimize the size of the device, there are practical limits to size reduction. For example, placing the sensor electrode and the infusion cannula too close together can reduce the accuracy of readings obtained by the sensor. As such, there may also be a minimum lateral separation between the infusion cannula and the sensor electrode. Additionally, to effectively deliver medicament and to obtain accurate sensor readings, as well as reliably retain the infusion cannula and sensor electrode within the user's skin, the infusion cannula and/or the sensor electrode should be inserted to a sufficient depth beneath the surface of the user's skin. Accordingly, there may be a minimum "travel" distance required by the insertion assembly to enable each of the infusion cannula and sensor electrode to penetrate the user's skin to a sufficient depth. Achieving these aspects (sufficient lateral separation and sufficient travel depth) can often lead to bulky and/or undesirably large devices.

In some embodiments, the insertion assembly can achieve both sufficient travel depth and sufficient lateral separation without requiring undue device size (e.g., without unduly increasing the height of the device). In various examples, the insertion assembly can include at least one actuator configured to drive first and second trocars that are releasably engaged with an infusion cannula and a sensor electrode, respectively. For example, the actuator can include a torsion spring. When the insertion assembly is actuated, the torsion spring rotates to cause the first and second trocars to fire axially (e.g., downwardly or otherwise outwardly from the device), thereby driving both the infusion cannula and the sensor electrode axially out of the device housing. By continued rotation of the torsion spring, the first and second trocars can be retracted upwardly into the device housing while the infusion cannula and sensor electrode remain in place inserted into the user's skin.

In some embodiments, the insertion assembly can include at least one epicyclic gear arrangement comprising an output to which a member (e.g., trocar) is coupled. The insertion assembly can, for example, include multiple epicyclic gear arrangements, such as a first epicyclic gear arrangement having a first output and a second epicyclic gear arrangement having a second output, where a first trocar is coupled to the first output and a second trocar is coupled to the second output. In such embodiments, actuation of the first epicyclic gear arrangement can drive linear travel of the first trocar and an associated sensor electrode coupled (e.g., movably coupled) to the first trocar. Similarly, actuation of the second epicyclic gear arrangement can drive linear travel of the second trocar and an associated infusion cannula coupled (e.g., movably coupled) to the second trocar. When the overall wearable device is arranged on the skin of a user, such actuation of the first and second epicyclic gear arrangements can advance the first trocar, the sensor electrode, the second trocar, and the infusion cannula into the skin of the user. Further actuation of the first and second epicyclic gear arrangements can, in some embodiments, cause retraction of the first trocar and second trocar from the skin of the user, while leaving the sensor electrode and the infusion cannula in the skin of the user.

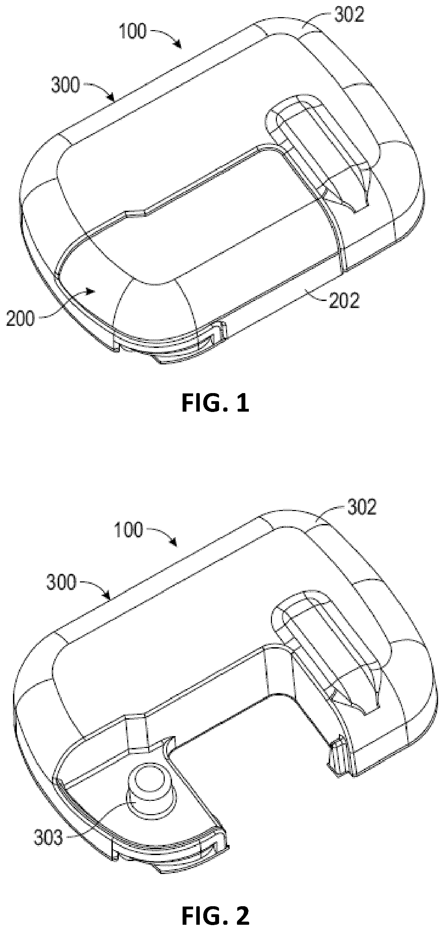

FIG. 1 is a perspective view of an infusion device with an integrated sensor ("device 100") that includes a durable assembly 200 and a disposable assembly 300 in accordance with several embodiments of the present technology. FIG. 2 illustrates the device 100 with the durable assembly 200 removed. In operation, a first side or surface (e.g., bottom side) of the device can be configured to be adhered to the user's skin with a second side or surface (e.g., top side) facing away from the user's skin. The device 100 includes the durable assembly 200 and the disposable assembly 300, each having respective housings 202 and 302. The durable assembly 200 and disposable assembly 300 can be secured to the skin of the user. For example, the durable assembly 200 and disposable assembly 300 can be disposed on an adhesive pad (not shown) with adhesive backing for securing to the user's skin. In various embodiments, the device 100 may have a length of about 35-60 mm; a width of about 30-45 mm; and an overall thickness or height of about 8-18 mm. Suitable housing materials include, but are not limited to, plastic or other materials having a modulus of elasticity of 0.2-1.0 million psi. In some embodiments, when positioned over the user's skin, a greatest height of the device over the user's skin is less than about 10 mm, less than 15 mm less than 20 mm, or less than 25 mm.

To use the infusion device 100, the user (e.g., a patient) connects the disposable assembly 300 to the durable assembly 200. Unless the reservoir of the disposable assembly 300 has been sufficiently pre-loaded, the user injects a desired amount of medicament into the reservoir via a fill port (e.g., disposed on a lower surface of the device 100). To adhere the device 100 to the user, an adhesive backing may be exposed on the bottom side of the device 100 and the device 100 can be applied to the skin surface. In some embodiments, the user then triggers insertion which causes both an infusion cannula and a sensor electrode to be advanced until at least their distal portions are beyond the housing 302 and inserted into the user's skin, as described in more detail below. Additionally or alternatively, insertion of the infusion cannula and the sensor electrode can be self-triggered by the device 100, such as in response to a self-generated signal. Examples of attachment of the device to a user and examples of use of the device are similar to that described in US Patent No. 10,159,786, assigned to Medtronic MiniMed, Inc., which is hereby incorporated by reference in its entirety.

The durable assembly 200 may include a housing 202, which at least partially covers one or more electronic components, such as a buzzer or other alarm device, one or more batteries or other energy supply, a microprocessor, a coil assembly (which functions as a motor stator) including one or more Hall-effect sensors, one or more wireless transceivers, and/or any other suitable components configured to power and/or control operation of the device 100. In some embodiments, the energy supply is a rechargeable battery, such as a rechargeable lithium-ion battery, which can, for example, store enough power to drive the motor continuously without needing a capacitor or other additional energy storage device.

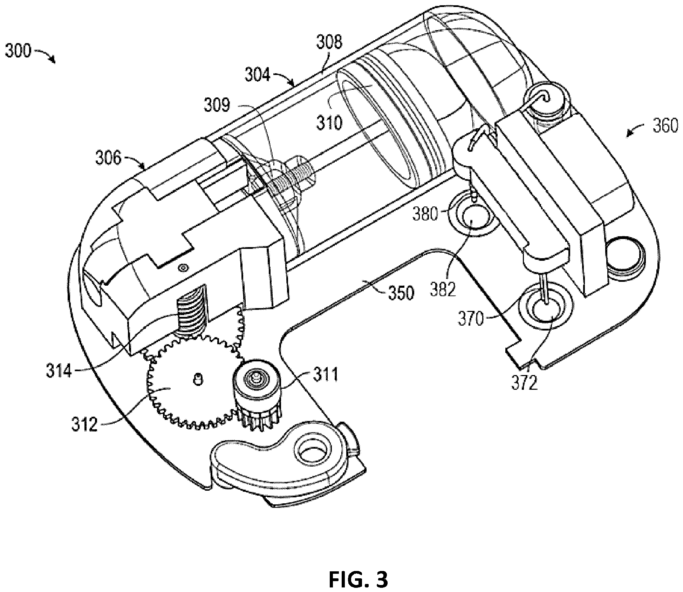

As shown in FIG. 2, the disposable assembly 300 includes a protrusion 303, over which a receptacle (not shown) in the durable assembly 200 is configured to fit such that the disposable assembly 300 and the durable assembly 200 are releasably mated together. The fit can, for example, include a mechanical engagement such as a snap fit assembly. However, in other embodiments, the durable assembly 200 can mate with the disposable assembly 300 via any suitable form-fit connection, mechanical interference, and/or in any suitable manner. It should be understood that although the protrusion 303 is shown as circular in FIG. 1B, it should be understood that the mating interface between the durable assembly 200 and the disposable assembly 300 can have any suitable form (e.g., ridges or ribs, etc.). The electronic components within the durable assembly 200 can wirelessly communicate with corresponding components within the disposable assembly 300. For example, with reference to FIG. 3, a magnetic stator within the durable assembly 200 can drive a magnetic motor rotor 311 within the disposable assembly 300, which in turn can drive a pusher 309 coupled to a plunger within a reservoir of the disposable assembly 300, e.g., using electromagnetic torque coupling, which is a coupling without direct mechanical coupling or electrical contact. These designs afford the additional advantage of being relatively simple to make waterproof, or at least water resistant. An example durable assembly 200 and disposable assembly 300 is more fully described in US Patent No. 10,159,786, assigned to Medtronic MiniMed, Inc., which is hereby incorporated by reference in its entirety.

Moreover, in some embodiments at least some of the components within the disposable assembly 300 can wirelessly communicate with component(s) within the durable assembly 200. For example, sensor electronics including electronic component(s) within the disposable assembly 300 can transmit data to electronic components within the durable assembly 200. For example, a continuous glucose monitor (CGM) within the disposable assembly 300 can collect low-level (e.g., millivolt) CGM signals that are conditioned (e.g., amplified, processed, digitized, etc.) within electronics of the disposable assembly 300. This pre-processed CGM data may then be wirelessly transmitted to electronic components within the durable assembly 200 for additional processing and/or for wireless transmission to one or more external devices (e.g., smartphone, tablet, etc.). Such transmission can include, for example, Bluetooth Low Energy (BLE) or other power-efficient methods for wirelessly transmitting the data. Because the disposable assembly 300 and the durable assembly 200 can be positioned so closely together, and because the transmission path is consistent and well-defined, the power required for transmission can be relatively low (as compared to systems in which a discrete CGM is positioned on an opposite side of a user's abdomen from a patch pump device). Additionally, by providing a power source (e.g., a battery 390) within the disposable assembly 300 that can be connected to one or more electronic components in the disposable assembly 300, this pre-processing and data communication between the durable assembly 200 and the disposable assembly 300 can be carried out with no physical electrical connections between the two.

The disposable assembly 300, shown in more detail in FIG. 3 with a portion of the housing 302 removed, may include a reservoir assembly 304 and an insertion assembly 360, each mounted on a baseplate 350. The reservoir assembly 304 can comprise a drive assembly 306, a reservoir 308, and a pusher 309 coupled to a plunger 310. The pusher 309 can be coupled to the drive assembly, and both the pusher 309 and the plunger 310 can be contained within the reservoir 308. The drive assembly 306 can comprise a magnetic motor rotor 311 and a gear train 312. The gear train 312 is coupled to the pusher which is positioned in the reservoir 308. The magnetic motor rotor 311 may be mechanically engaged with the gear train 312 to affect translation of the pusher 309 and the plunger 310 within the reservoir 308. In operation, translation of the pusher 309 and plunger 310 within the reservoir (e.g., via actuation of the magnetic motor rotor 311) causes fluid (e.g., medicament) to be driven out of the reservoir 308 and through an infusion cannula to be delivered to a user at a subcutaneous treatment site.

The reservoir 308 may be prefilled with a medicament. The medicament, for example, can be U-100 insulin or U-500 insulin or other concentrations of insulin to suit different user dose or use profiles. The reservoir 308 may be user-fillable by way of a fill port (not shown). In those instances where the reservoir 308 is filled by the user, the user may completely fill the reservoir to capacity with medicament, or the user may choose to introduce less medicament and not completely fill the reservoir. Since an unknown amount of medicament may be injected into a user-filled reservoir, a plunger-pusher zeroing procedure (or "plunger seek") may be user-initiated or may be an automatic aspect of pump operation. A plunger seek procedure precisely determines and/or sets, before any medicament dispensing, exactly how far the pusher travels before it engages the plunger, enabling a calculation to determine the amount of medicament in the reservoir and, therefore, an estimate of time-to-empty and time for disposable assembly replacement. Additional details regarding the plunger seek procedure can be found in commonly owned U.S. Patent Publication No. US 2022/0143305 entitled "Remotely activated cannula insertion", which is hereby incorporated by reference in its entirety.

The insertion assembly 360 can be configured to insert both an infusion cannula 380 and a sensor electrode 370 out of the housing 302 via a first port 382 and a second port 372, respectively. The insertion assembly 360 can also include one or more trocars with sharpened or pointed distal ends configured to puncture skin of the user and facilitate insertion of the infusion cannula 380 and the sensor electrode 370 in the punctured skin. Once inserted into the user's skin, the infusion cannula 380, which is fluidically coupled to the reservoir 308, can deliver medicament (e.g., insulin) therethrough to the user. Additionally, once the sensor electrode 370 is inserted into the user's skin, the sensor electrode can detect one or more physiological parameters, such as glucose levels in the intradermal and/or subcutaneous space. As will be known to one of ordinary skill in the art, measurements of glucose obtained in intradermal and/or subcutaneous space can be used to derive or infer blood glucose levels of the user. Such physiological parameters obtained via the sensor electrode 370 can optionally be used to control delivery of medicament via the infusion cannula 380. For example, as a user's glucose level rises above a predetermined threshold (as determined based at least in part on measurements obtained via the sensor electrode 370), the magnetic motor rotor 311 can be initiated (e.g., by the electronics in the durable assembly 200) to drive the plunger 310 an appropriate amount to dispense medicament from the reservoir 308 into the user's body via the infusion cannula 380.

### II. Epicyclic insertion assembly

Generally, an insertion assembly in an infusion device (e.g., infusion device with an integrated sensor) in accordance with the present technology functions to actuate insertion of one or more trocars, an infusion cannula, and a sensor electrode into a user's body. For example, an insertion assembly in an infusion device can include at least one epicyclic gear arrangement that includes a ring gear and a planet gear that are mounted such that the center of the planet gear revolves around the center of the ring gear. The planet gear is engaged with the ring gear (e.g., the ring gear and planet gear have respective teeth that mesh with each other) such that the planet gear rolls on the inside of the pitch circle of the ring gear. The overall epicyclic gear arrangement can have an output (e.g., on the planet gear) to which a member (e.g., a piercing member, such as a trocar movably coupled to or movably engaged with an instrument such as a sensor electrode or infusion cannula) is coupled. The epicyclic gear arrangement can be configured such that the member can be restrained to travel in a linear path, which in turn ensures that actuation of the epicyclic gear arrangement drives travel of the trocar and the instrument along the linear path.

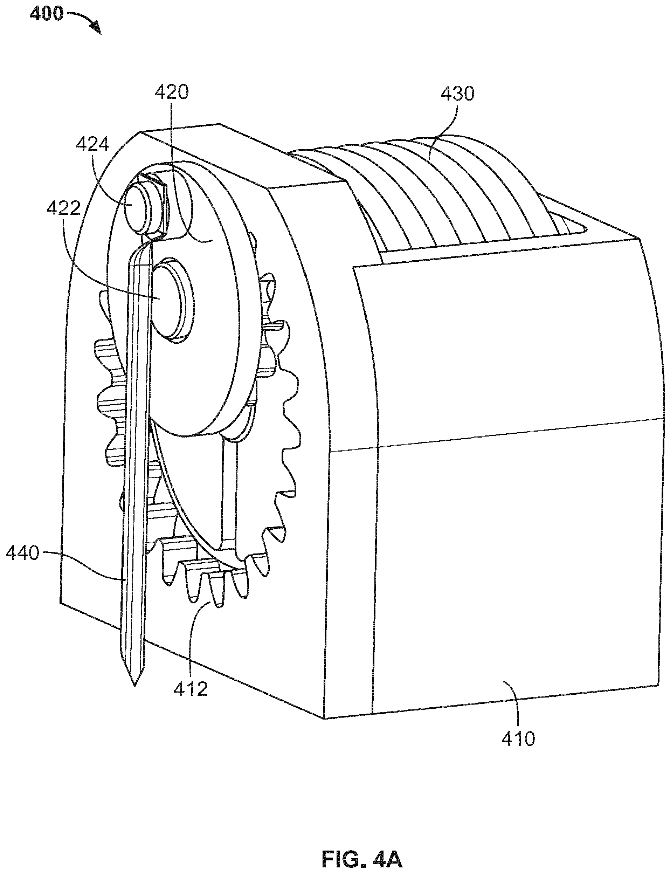

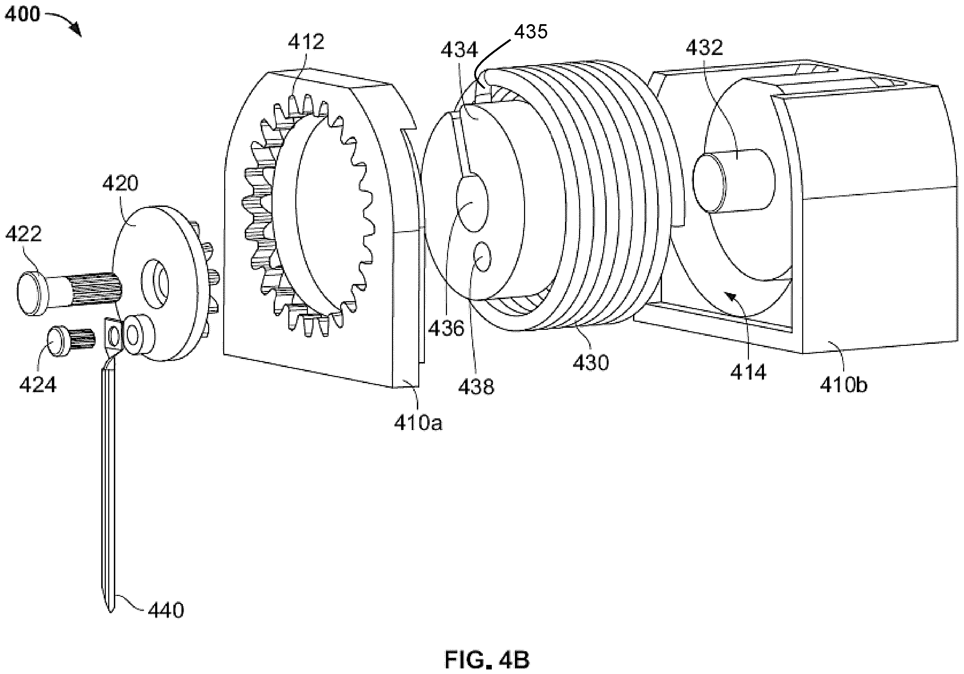

An example insertion assembly with an epicyclic gear arrangement is shown in FIGS. 4A and 4B as an insertion assembly 400. The epicyclic gear insertion assembly 400 can take the place of the insertion assembly 360 described above with reference to FIG. 3. The insertion assembly 400 includes a housing 410 that at least partially houses an actuator (e.g., torsion spring 430) and a hub 434 that is coupled to the actuator such that the hub 434 can function as a drive shaft input to the epicyclic gear arrangement. A piercing member 440 (e.g., a trocar) can be coupled to an output of the epicyclic gear arrangement so as to be driven or advanced in a suitable manner by the epicyclic gear arrangement, as further described herein.

As shown in FIG. 4B, the housing 410 may include multiple components coupled to one another (e.g., a first housing portion 410a coupled to a second housing portion 410b), such as via one or more fasteners (e.g., mechanical fasteners, epoxy, etc.) and/or mechanical interfit (e.g., snap fit). Although the housing 410 is depicted as including two separate housing portions, it should be understood that in some embodiments, the housing 410 may include fewer than two (e.g., a single integrated component) or more than two (e.g., three, four or more, etc.).

As shown in FIG. 4B, the housing (e.g., housing portion 410b) can include a recess 414 that is sized and shaped to receive the hub 434, and a bearing shaft 432 mounted in the recess. The hub 434 can include an aperture 436 that engages with the bearing shaft 432 such that the hub 434 is rotatable around the bearing shaft 432. The actuator is coupled to the hub 434 so as such that the actuator can drive rotation of the hub 434 around the bearing shaft 432. For example, the torsion spring 430 can be a helical spring that includes a first end coupled to the housing 410 (e.g., housing portion 410b) and a second end coupled to the hub 434, such as via one or more crank pins 435. The torsion spring 430 can be mounted over the hub 434 such that the longitudinal axis of the torsion spring 430 is aligned (e.g., coincident) with the axis around which the hub 434 rotates on the bearing shaft 432.

The epicyclic gear arrangement can include a ring gear 412 and a planetary gear 420 that is engaged with the ring gear 412. As shown in FIG. 4B, the ring gear 412 can be formed into a surface of the housing (e.g., part of the housing portion 410a), although in some embodiments the ring gear 412 can be formed separately from the housing and coupled to the housing (e.g., fixedly coupled via mechanical fasteners, epoxy, and/or the like). The ring gear 412 can be coaxial with the hub 434 and/or bearing shaft 432. The rotational axis of the planetary gear 420 can be offset from the center of the ring gear 412 in an epicyclic manner.

The hub 434 can provide driving input to the epicyclic gear arrangement through a crank pin 422 located in a crank pin hole 438 that is offset from the rotational axes of the hub 434 and the bearing shaft 432, thereby forming a crankshaft. For example, planetary gear 420 can be coupled to the hub 434 via the crank pin 422, such that rotation of the hub 434 causes the planetary gear 420 to travel around the center of the ring gear 412 with the external teeth of the planetary gear 420 engaging the internal teeth of the ring gear 412.

The piercing member 440 can be coupled to an output of the epicyclic gear arrangement. For example, the piercing member 440 (e.g., trocar) can be pivotably coupled to the planetary gear 420, such as via a pivot pin 424, such that the piercing member 440 can rotate around the pivot pin 424. The axis of the pivot pin 424 can be offset from the axis of the crank pin, thereby forming a second crankshaft. Geometrically, it can be shown that the path of the pivot pin 424 can be designed in a linear (vertical, in the assembly orientation shown in FIGS. 4A and 4B) path when the amount of offset between the rotational axis of the hub 434 and the crank pin 422 is about twice the amount of offset between the crank pin 422 and the pivot pin 424.

The piercing member 440 can also be constrained to move along a restricted path (e.g., a linear path along a longitudinal axis of the piercing member 440) such that rotation of the planetary gear 420 causes the piercing member 440 to travel along the restricted path. In some embodiments, other physical components (not shown) or features of the housing 410 can be configured to constrain the travel of the piercing member 440 to the restricted path. Examples of path-constraining physical components or features are further described herein.

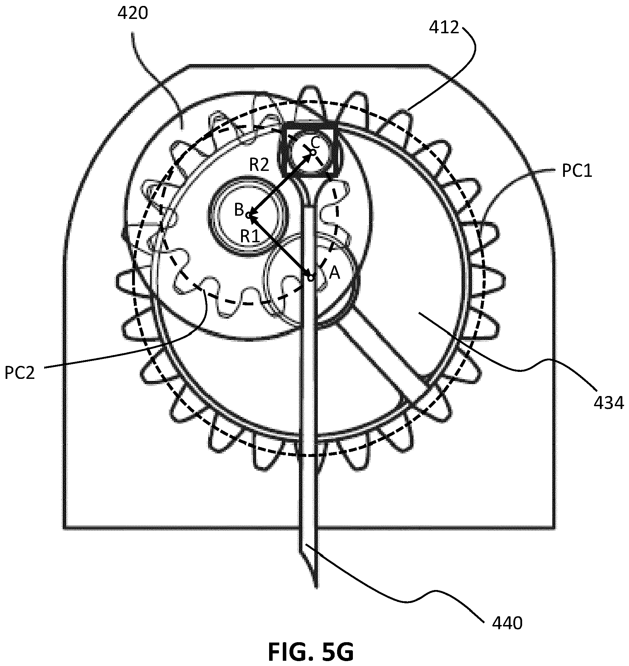

In an assembled configuration such as that as shown in FIG. 4A, the torsion spring 430 can be torqued into a loaded, compressed state in which the torsion spring 430 is biased to unwind, thereby causing rotation of the hub 434 (or alternatively, the torsion spring 430 can be torqued into a loaded, expanded state in which the torsion spring 430 is biased to wind, thereby causing rotation of the hub 434). In either loaded state, the torsion spring 430 can be releasably locked (e.g., in a cocked configuration), such that rotational movement is prohibited until a trigger (as further described herein) is activated. For example, a manual trigger (e.g., depressing a button or lever), or a remote trigger (e.g., a wireless signal sent to an actuatable release mechanism) can release the torsion spring 430 and enable rotational movement of the torsion spring 430 to cause rotation of the hub 434. This rotation of the hub 434 drives, through the crank pin 422 engaged with crank pin hole 438 in the hub 434, rotational movement of the planetary gear 420 around the center axis of the planetary gear 420, as well as revolving movement of the planetary gear 420 around the center axis of the ring gear 412. For example, as shown in the schematic of FIG. 5G, such rotation of the hub 434 drives rotational movement of the planetary gear 420 around its center of rotation B, as well as revolving movement of the planetary gear 420 around the center axis A of the ring gear 412. These movements of the planetary gear 420, in turn, causes movement of the piercing member 440 along its constrained linear path.

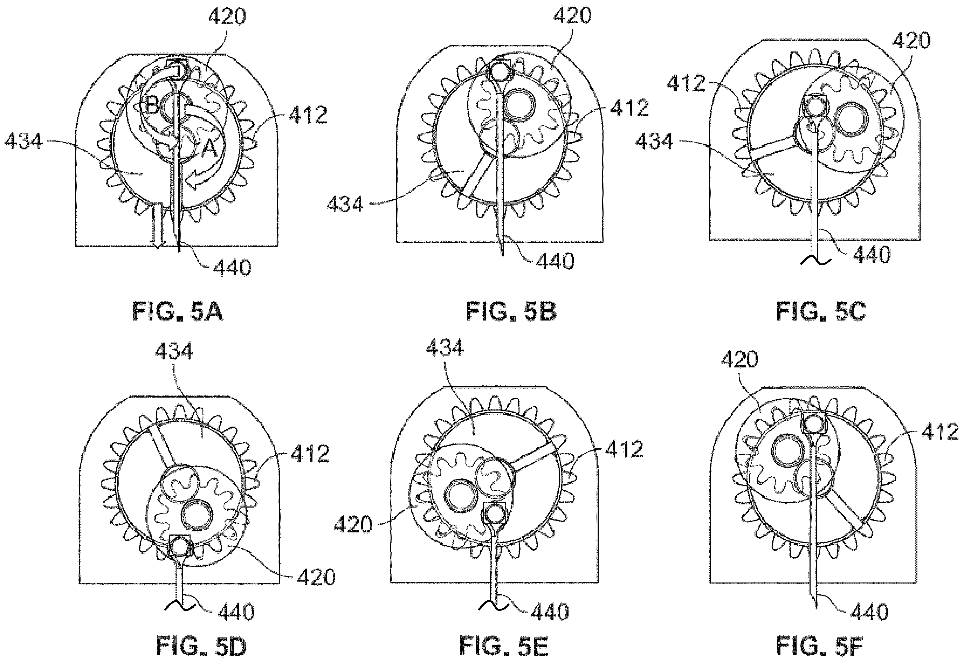

FIGS. 5A-5F illustrate, in more detail, an example operation of the epicyclic gear arrangement shown in FIGS. 4A and 4B for driving the piercing member 440 along a linear path (e.g., up and down vertically, in the assembly orientation shown in FIGS. 5A-5F). FIG. 5A depicts the epicyclic gear arrangement in an "UP" position in which the piercing member 440 is in a fully retracted state relative to the housing 410. Actuation of the torsion spring (not shown) can cause the hub 434 to rotate in a first direction A (e.g., clockwise) which results in the planetary gear 420 to rotate in a second direction B that is opposite direction A (e.g., counter-clockwise). FIG. 5B depicts the epicyclic gear arrangement after the hub 434 has rotated 30 degrees in direction A. With such rotation, the planetary gear 420 has similarly rotated about 30 degrees but in direction B about its rotational axis, and also traveled within the ring gear 412 around the rotational axis of the hub 434. This movement of the planetary gear 420 has also caused the piercing member 440 to advance or travel downwards a small amount. FIGS. 5C and 5D depict the epicyclic gear arrangement when the hub 434 has further rotated to about 75 degrees and about 160 degrees, respectively. In each of FIGS. 5C and 5D, the piercing member 440 has traveled progressively farther downwards with such further rotation until it is in (or near) a "DOWN" position as shown in FIG. 5D, in which the piercing member 440 is in a fully extended or outwards state relative to the housing.

FIGS. 5E and 5F depict the epicyclic gear arrangement when the hub 434 has further rotated to about 250 degrees and about 320 degrees, respectively. In each of FIGS. 5E and 5F, the piercing member 440 has traveled progressively farther upwards with such further rotation until it returned to be in (or near) the "UP" position.

Accordingly, actuation of the torsion spring can cause the hub 434 to rotate a first amount to travel from the "UP" position to the "DOWN" position, and further actuation of the torsion spring can cause the hub 434 to rotate a second amount beyond the first amount to travel from the "DOWN" position to the "UP" position. In some embodiments, the piercing member 440 is a trocar that is configured to be inserted into skin of a user and movably coupled to an instrument (e.g., sensor electrode, infusion cannula, etc.). In these embodiments, the "UP" position can correspond to a state where the trocar and/or the instrument are retracted so as to be disposed within the housing, and the "DOWN" position can correspond to a state where the trocar and/or the instrument are extended at least partially beyond the housing for insertion into the user's skin.

Specific amounts of rotation for achieving the "UP" and "DOWN" positions may depend on, for example, the relative sizes (e.g., pitch, diameter, etc.) of the ring gear 412 and/or planetary gear 420, which may vary according to a desired amount of travel of the piercing member 440. FIG. 5G is a schematic of various gear relationships in an example epicyclic gear arrangement including a ring gear 412 and a planetary gear 420 engaged with the ring gear 412. The ring gear 412 has a pitch circle PC1 that is the theoretical rolling contact circle for the ring gear, and the planetary gear 420 has a pitch circle PC2 that is the theoretical rolling contact circle for the planetary gear 420. Pitch circles PC1 and PC2 are in theoretical contact, with involute teeth on the ring gear 412 and the planetary gear 420 arranged on PC1 and PC2, respectively, so as to maintain ideal gear contact with each other for suitable internal gear mesh. The total stroke length or travel of the piercing member 440 is defined by the size of the ring gear 412 pitch circle PC1 (e.g., equal to the diameter of the ring gear 412 pitch circle PC1).

Also shown in FIG. 5G is the main crank radius R1, which is the distance between the center of rotation B of the planetary gear 420 and the rotating input axis of the epicyclic gear arrangement (e.g., the rotational axis of the hub 434, which is coaxial with the center axis A of the ring gear 412). R2 is the planetary gear crank radius (the distance between the center of rotation C of the piercing member 440 and the center of rotation B of the planetary gear 420).

For a linear travel mechanism, the planetary gear pitch circle PC2 is half the size of the ring gear pitch circle PC1 (i.e., PC1 = 2 x PC2), and the main crank radius R1 and planetary gear crank radius R2 are equal (i.e., R1 = R2). This also means R1 and R2 are each a quarter of the ring gear pitch circle PC1 (i.e., R1 = PC1 / 4 and R2 = PC1 / 4).

In some example embodiments, an integrated sensor within an infusion device has a desired sensor electrode stroke length of about 11.5 mm. To accommodate this, the sensor electrode can be inserted via an epicyclic insertion assembly that includes dimensions as shown in Table 1 below.

**Table 1. Dimensions of an example epicyclic gear arrangement providing a linear stroke length output of 11.5 mm.**

| **Epicyclic Gear Arrangement Feature** | **Dimension** |
|---|---|
| Ring gear pitch circle (PC1) diameter | 11.5 mm |
| Planetary gear pitch circle (PC2) diameter | 5.75 mm |
| Main crank radius (R1) | 2.875 mm |
| Planetary gear crank radius (R2) | 2.875 mm |

Additionally or alternatively, in some example embodiments, an infusion device has a desired infusion cannula stroke length of about 8 mm. To accommodate this, the infusion cannula can be inserted via an epicyclic insertion assembly that includes dimensions as shown in Table 2 below.

**Table 2. Dimensions of an example epicyclic gear arrangement providing a linear stroke length output of 8.0 mm.**

| **Epicyclic Gear Arrangement Feature** | **Dimension** |
|---|---|
| Ring gear pitch circle (PC1) diameter | 8.0 mm |
| Planetary gear pitch circle (PC2) diameter | 4.0 mm |
| Main crank radius (R1) | 2.0 mm |
| Planetary gear crank radius (R2) | 2.0 mm |

During operation of the epicyclic gear arrangement, the actuating load (e.g., spring load from a torsion spring) is partially transferred through the gear teeth. As such, it may be advantageous to design the actual gear tooth profile (on the ring gear 412, the planetary gear 420, etc.) as large as possible to reduce tooth bending stress. In the example shown in FIG. 5G, the gear tooth profiles are sized large while maintaining a planetary tooth count above 8, and the gears have a module near about 0.5. However, it should be understood that in some embodiments, the various features of the ring gear and/or planetary gear may vary in shape and/or size (e.g., depending on the specific application of the insertion assembly).

In some embodiments, an epicyclic insertion assembly such as that described herein advantageously does not generate eccentric loads that need to be resisted with multiple sliding bearings. Accordingly, this mechanism is efficient in maintaining a good stroke to size ratio, thereby enabling use of a smaller actuator (e.g., torsion spring) to provide a given desired insertion force. Including a smaller actuator can thus lower the overall size of a device (e.g., infusion device with an integrated sensor) including such an epicyclic insertion assembly.

In some embodiments, an infusion device with an integrated sensor can include an insertion assembly that includes multiple epicyclic gear arrangements. One or more of such multiple epicyclic gear arrangements can operate on similar principles as the epicyclic gear arrangement described herein with respect to FIGS. 4A-5G. In some embodiments, an insertion assembly can include two epicyclic gear arrangements. For example, an insertion assembly can include a first epicyclic gear arrangement for controlling travel of a first trocar to which a sensor electrode is movably coupled or movably engaged. In some embodiments, the first epicyclic gear arrangement functions to move the first trocar and/or sensor electrode from an unfired state, through one or more inserted states, and to a fully fired state. Furthermore, the insertion assembly can also include a second epicyclic gear arrangement for controlling travel of a second trocar to which an infusion cannula is movably coupled or movably engaged. In some embodiments, the second epicyclic gear arrangement functions to move the second trocar and/or the infusion cannula from an unfired state, through one or more inserted states, and to a fully fired state.

As used herein with respect to the first and second epicyclic arrangements, an "unfired state" can refer to a state in which the epicyclic arrangement has not yet moved the trocar and associated instrument, such as a sensor electrode or infusion cannula, out of the housing and into a user's skin. An "inserted state" can refer to an intermediate position in which the trocar and/or instrument has been moved downwardly, out of the housing, and into the user's skin. A "fully fired state" can refer to a terminal position in which the epicyclic arrangement has retracted the trocar upwardly and out of the user's body while leaving the instrument extending beyond the housing and/or into the user's body.

In some embodiments, the relative positions of the first and second epicyclic arrangements in the insertion assembly can be selected based at least in part on desired relative positions of the sensor electrode and the infusion cannula. In various examples, the lateral distance between the sensor electrode and the infusion cannula can be selected to provide appropriate performance once inserted within the user's skin. If the lateral distance is too small, then the medicament provided via the infusion cannula may interfere with physiological measurements obtained via the sensor electrode. On the other hand, having too great a lateral separation may lead to the infusion device with an integrated sensor being undesirably large and bulky. In various embodiments, the lateral separation between the sensor electrode and the infusion cannula can be at least about 10 mm, at least about 15 mm, or at least about 20 mm. In some specific embodiments, the infusion cannula and the sensor electrode are laterally spaced apart from one another by about 15 mm.

Furthermore, the relative sizes and/or strokes provided by the first and second epicyclic arrangements in the insertion assembly can be selected based at least in part on desired insertion depths for the sensor electrode and the infusion cannula. In some embodiments, it may be desirable to insert the sensor electrode and the infusion cannula to a certain depth beneath the surface of the user's skin. If the sensor electrode is not sufficiently deep, the physiological measurements may be unreliable. Additionally or alternatively, if the infusion cannula is not sufficiently deep, the medicament may not be effectively delivered to the user. In various embodiments, the insertion depth (e.g., the distance from the further tip of the sensor electrode and/or the infusion cannula with respect to a lower surface of the housing when in the inserted state) may be at least about 5mm-10 mm or more. The insertion depth of the sensor electrode may be equal to the insertion depth of the infusion cannula, or the insertion depth of the sensor electrode may be different than the insertion depth of the infusion cannula. For example, in some specific embodiments, the stroke length of the first epicyclic gear arrangement can be about 11.5 mm so as to provide an insertion depth of the sensor electrode of about 11.5 mm). Furthermore, in some embodiments, the stroke length of the second epicyclic gear arrangement can be at least about 7 mm (e.g., about 7 mm or about 8.5 mm) so as to provide an insertion depth of the infusion cannula of at least about 7 mm (e.g., about 7 mm or about 8.5 mm). However, these specific examples are not limiting and other stroke lengths and insertion depths are contemplated.

Achieving both sufficient lateral separation and sufficient insertion depth presents certain design challenges, particularly while trying to maintain a compact form factor for the infusion device with an integrated sensor. Various embodiments of the insertion assembly disclosed herein may achieve both the desired lateral separation and the desired insertion depth for the infusion cannula and the sensor electrode, even when the desired depths of insertion may be different for the infusion cannula and the sensor electrode.

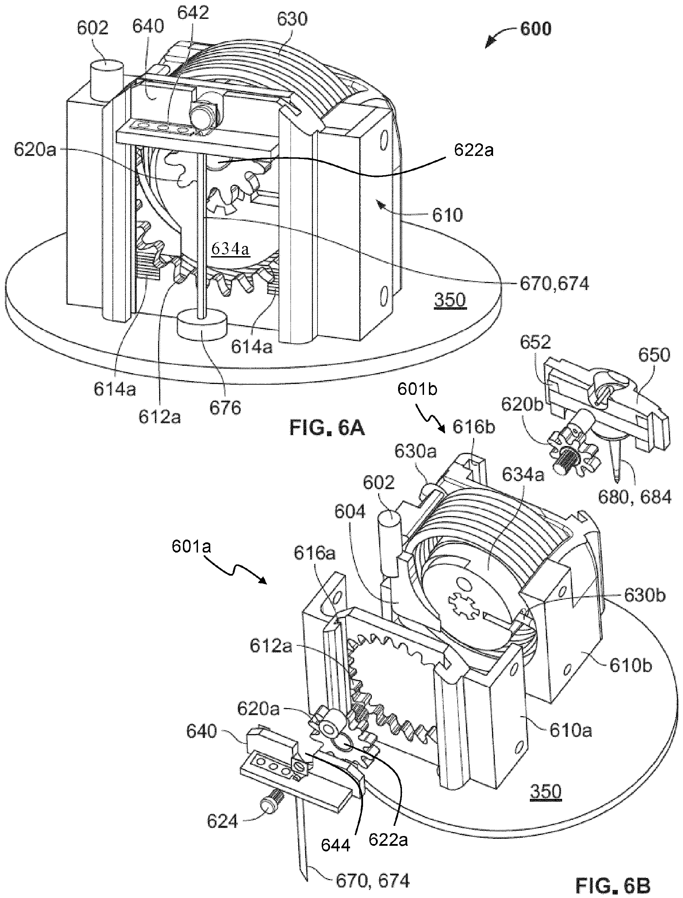

FIGS. 6A-7B depict an example insertion assembly 600 that includes a first epicyclic gear arrangement 601a for controlling travel of a first trocar 674 (and/or a sensor electrode 670 movably coupled to the first trocar 674), and a second epicyclic gear arrangement 601b for controlling travel of a second trocar 684 (and/or an infusion cannula 680 movably coupled to the second trocar 684). In some embodiments, the sensor electrode 670 can be arranged within a lumen of the first trocar 674, and/or the second trocar 684 can be arranged within a lumen of the infusion cannula 680. The first and second epicyclic arrangements 601a and 601b can be positioned on opposite sides of the insertion assembly 600 (e.g., back-to-back), such that the sensor electrode 670 and the infusion cannula 680 are spaced apart. In some embodiments, the first epicyclic gear arrangement 601a functions to move the first trocar and/or sensor electrode from an unfired state, through one or more inserted states, and to a fully fired state. Similarly, in some embodiments, the second epicyclic gear arrangement 601b functions to move the second trocar and/or the infusion cannula from an unfired state, through one or more inserted states, and to a fully fired state. In some embodiments as described in further detail herein, the first and second epicyclic arrangements 601a and 601b can be driven simultaneously (e.g., in tandem) by a single actuator so as to cause simultaneous movement of the first trocar and second trocar in parallel.

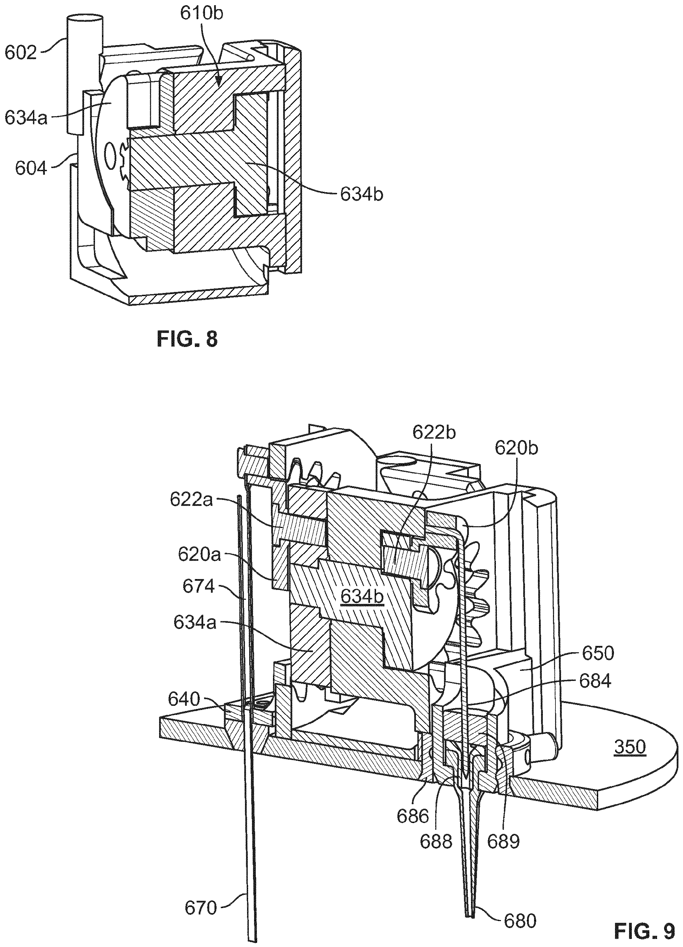

As shown in FIGS. 6A and 6B, the insertion assembly 600 can include a housing 610 that supports a hub 634a rotatable on a bearing shaft (not shown). Similar to the housing 410, the housing 610 can include a first housing portion 610a and a second housing portion 610b that can be coupled together. A torsion spring 630 or other actuator can be configured to rotate the hub 634a, similar to torsion spring 430. For example, as shown in FIG. 6B, the torsion spring 630 can include a first spring end 630a that couples to the second housing portion 610b, and a second spring end 630b that couples to the hub 634a (e.g., via one or more fasteners, epoxy, mechanical engagement, etc.). The torsion spring 630 can be coupled to the housing and the hub in a loaded state (e.g., cocked to a compressed state), and maintained in the loaded state with a releasable trigger mechanism 604. The trigger mechanism 604 can include a sliding latch that selectively engages a recess or other latching feature of the hub 634a, and can be controlled by a release (e.g., button 602). For example, a depression of button 602 can cause the trigger mechanism 604 to slide downwards (in the orientation shown in FIG. 6B and FIG. 8), thereby disengaging the latch from the hub 634a and releasing the torsion spring 630 from its loaded state. Accordingly, activation of the trigger mechanism 604 can cause the torsion spring 630 to rotate the hub 634a. Although the trigger mechanism 604 is shown to include a sliding latch selectively controlled by a button 602, it should be understood that other embodiments can additionally or alternatively include other trigger mechanisms, such as pins, swinging latches, and/or the like. In various examples, the latch can be manually triggered (e.g., via a mechanical button or other actuator), or may be remotely triggered (e.g., via an electrical signal provided by a controller to release the latch).

Like the epicyclic arrangement described herein with respect to FIGS. 4A and 4B, the first epicyclic arrangement 601a shown in FIGS. 6A and 6B can include a ring gear 612a (e.g., defined in the first housing portion 610a) and a planetary gear 620a that is engaged with the ring gear 612a. In one example, the ring gear 612a and the planetary gear 620a can have a module of about 0.5 (e.g., 0.4792) and a tooth count of 24 and 12, respectively. The ring gear 612a can be coaxial with a hub 634a. The planetary gear 620a can have a rotational axis that is offset from the rotational axis of the hub 634a, and can be coupled to the hub 634a via a crank pin 622a. The first trocar 674 can be pivotably coupled to the planetary gear 620a, for example via a pivot pin 624, via a bent trocar portion similar to that described herein for the second trocar 684, or in any suitable manner, and define a recess or lumen. The sensor electrode 670 can be movably coupled to the first trocar 674, such as by being inserted in the recess or lumen of the first trocar 674. For example, in some embodiments, the first trocar 674 can have a C-shaped cross-section along at least a portion of its length, so as to provide a trough-like recess for holding the sensor electrode 670. As shown in FIG. 9, for example, the sensor electrode 670 and the first trocar 674 can be coaxial with each other (e.g., the sensor electrode 670 arranged telescopically within the first trocar 674). The first trocar 674 can have a sharpened or pointed distal end and made of metal or another suitable rigid material, such that during an insertion process, the first trocar 674 can help puncture skin of a user and enable placement of the sensor electrode 670 in the punctured skin.

Motion of the first trocar 674, and of the sensor electrode 670 that is coaxial with the first trocar 674, can be constrained to a linear path at least in part by a sensor carrier 640. For example, the sensor electrode 670 can be coupled to the sensor carrier 640, and the sensor carrier 640 can be slidingly engaged with the housing 610 (e.g., the sensor carrier 640 can include tongue feature(s) that are engaged within a grooved track 616a in the first housing portion 610a, or vice versa), such that motion of the sensor carrier 640 is generally limited along one axis aligned with the insertion direction of the first trocar 674 and the sensor electrode 670.

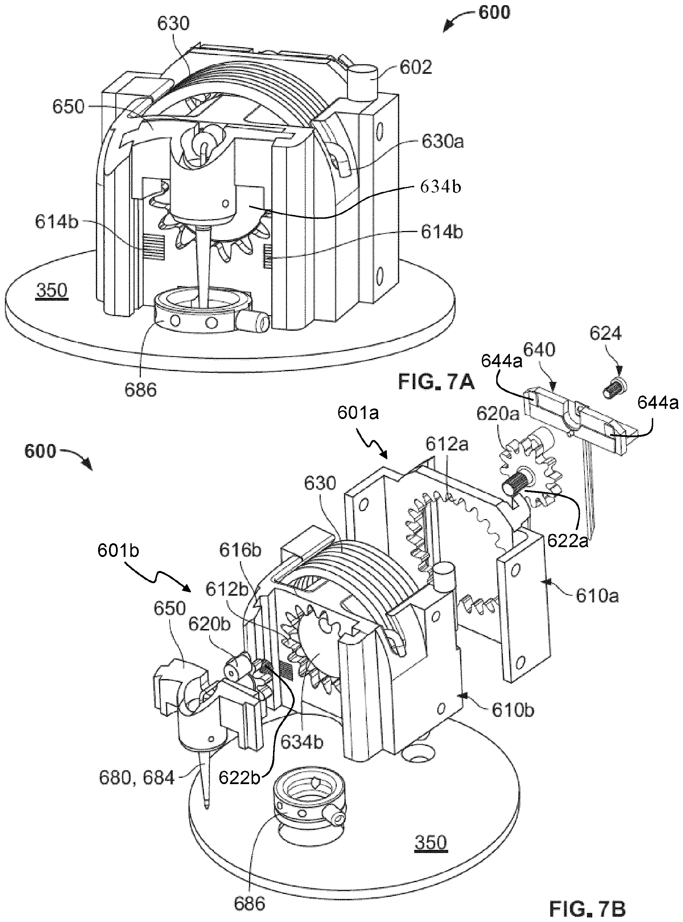

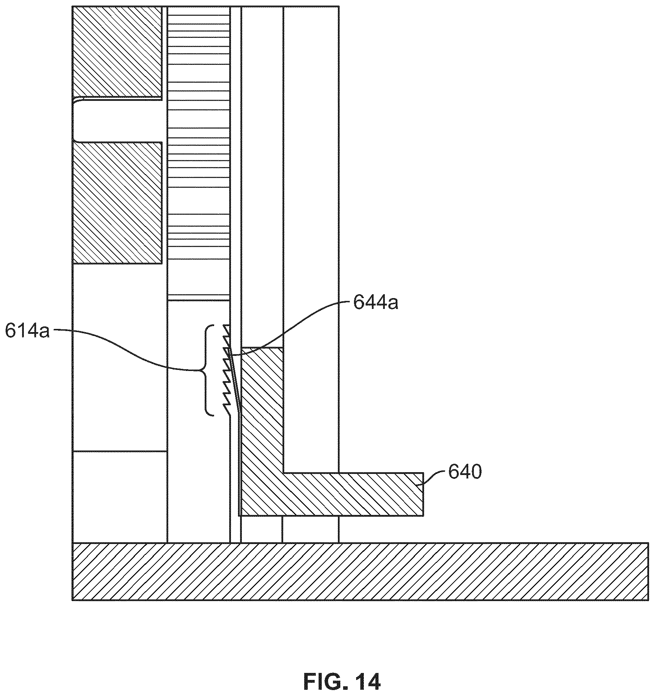

In some embodiments, the insertion assembly can further include one or more latches configured to selectively limit a position of the sensor carrier 640 relative to the housing 610. For example, the insertion assembly can include a one-way latch that, when engaged, holds the sensor carrier 640 in or near a fully inserted state, thereby holding the sensor electrode 670 in or near the fully inserted state. In some embodiments, the one-way latch includes one or more ratchet features. For example, as shown in FIG. 7B, a backside or other surface of the sensor carrier facing the housing 610 can include one or more flexible arms 644a (e.g., one arm on each side of the sensor carrier 640). Each flexible arm 644a can have a bent free end that is configured to engage lateral latch grooves 614a on the housing (latch grooves 614a shown in FIG. 6A). A detailed view of this latch engagement is shown in FIG. 14A. When the sensor carrier 640 moves downward a sufficient distance for the arms 644a to engage the lateral latch grooves 614a, the sensor carrier 640 is substantially prevented from reverse travel in the upward direction even if the hub 634a is further rotated, thereby substantially keeping the sensor electrode 670 (coupled to the sensor carrier 640) in the downward (e.g., inserted) position. However, after the arms 644a are engaged with the lateral latch grooves 614a, further rotation of the hub 634a can cause the first trocar 674 (coupled to the planetary gear 620a) to retract and travel in the upward direction. In other words, the one or more latches are configured to selectively hold the sensor carrier 640 and the sensor electrode 670 in a fully inserted state, but do not restrain the removal of the first trocar 674 from the fully inserted state. Accordingly, such one or more latches enables insertion of the sensor electrode 670 into a user's skin while facilitating subsequent removal of the first trocar 674 from the user's skin. Although a ratcheting latch is shown in the figures, it should be understood that in some embodiments, the epicyclic insertion assembly can additionally or alternatively include other types of latches or one-way securement mechanisms (e.g., cantilevered bars that mate with a feature of the housing 610 and/or device housing 350, adhesive, suitable contact-based fasteners such as hook-and-loop fasteners, etc.).

In some embodiments, the sensor electrode 670 can include (e.g., at its proximal end) sensor electronics 642, which can include one or more electronic components configured to facilitate obtaining physiological measurements via the sensor electrode 670. The sensor electronics 642 can be powered at least in part by a battery (e.g., similar to battery 390 as described herein with respect to FIG. 3). The sensor electronics 642 can include, for example, suitable analog and/or digital components, circuitry, processors, transceivers, etc. The sensor electronics 642 can be arranged, for example, on one or more suitable printed circuit boards and configured to receive signals from the sensor electrode 670. In some embodiments, as shown in FIGS. 6A and 6B, at least a portion of the sensor electronics 642 can be coupled to the sensor carrier 640 (e.g., with epoxy) so as to move with the sensor electrode 670 as the sensor carrier 640 travels in response to actuation by the epicyclic gear arrangement.

As shown in FIGS. 6A and 6B, a proximal portion of the sensor electrode 670 can be coupled to a first (e.g., lower, in the depicted device orientation) surface of the sensor carrier 640, and the sensor electrode 670 can extend downwardly toward the housing 350 of the infusion device, such as through an aperture or other suitable cutout of the sensor carrier 640. The first trocar 670 (arranged telescopically around the sensor electrode 670) can also extend downwardly through the same aperture, but is not fixedly coupled to the sensor carrier 640 and can instead freely axially pass through the aperture in the sensor carrier 640 along the insertion direction. The first trocar 670 can also extend through a septum 676 on the device housing 350. The septum 676 can function to guide the first trocar 670 during its linear travel and/or seal the sensor electrode 670 against fluid ingress once the sensor electrode 670 is disengaged from the first trocar 674. The septum 676 can, for example, include a suitable elastomeric material.

A proximal portion of the first trocar 670 can be pivotably coupled to the planetary gear 620a, such as with a pivot pin 624. As shown in FIG. 6B, a second hole or cutout 644 on a second (e.g., vertical) surface of the sensor carrier 640 can accommodate the connection point for the pivot pin 624 coupling the first trocar 674 to the planetary gear 620a. While the sensor carrier 640 can have an L-shaped cross-section as shown in FIGS. 6A and 6B, it should be understood that the sensor carrier 640 can alternatively have other suitable shape to accommodate the downward passage of the first trocar 674 and sensor electrode 670, and the pivoting connection of the first trocar 674 to the planetary gear 620a.

Like the epicyclic arrangement described herein with respect to FIGS. 4A and 4B, the second epicyclic arrangement 601b shown in FIGS. 7A and 7B can include a ring gear 612b (e.g., defined in the second housing portion 610b) and a planetary gear 620b that is engaged with the ring gear 612b. In one example, the ring gear 612b and the planetary gear 620b can have a module of about 0.5 (e.g., 0.4792) and a tooth count of 16 and 8, respectively. The ring gear 612b can be coaxial with a hub 634b. The planetary gear 620b can have a rotational axis that is offset from the rotational axis of the hub 634b, and can be coupled to the hub 634b via a crank pin 622b.

As shown in the cross-sectional views depicted in FIGS. 8 and 9, the hub 634a for driving the first epicyclic gear arrangement and the hub 634b for driving the second epicyclic gear arrangement can be coupled to each other. For example, the hubs 634a and 634b can be splined together (e.g., with one or more keys), or have any other suitable mating features to facilitate fixed coupling to one another. As shown in FIGS. 7A-7B, the same actuator (e.g., torsion spring 630) can be coupled to the hub 634a so as to actuate both hubs 634a and 634b. In this manner, the coupled hubs 634a and 634b can function as a single driveshaft for driving the first and second epicyclic gear arrangements (and controlling travel of the first and second trocars) in tandem. Alternatively, the same actuator (e.g., torsion spring 630) can be coupled to the hub 634b so as to actuate both hubs 634a and 634b. In some embodiments, however, the hubs 634a and 634b can be actuated independently by separate actuators. Furthermore, in some embodiments, instead of each of the first and second epicyclic arrangements 601a and 601b having respective separate hubs 634a and 634b coupled to each other, the hubs 634a and 634b can be integrally formed together as a single hub. The torsion spring 630 can be coupled to the single hub such that the single hub commonly drives the motion of the planetary gears 620a and 620b in the first and second epicyclic gear arrangements, respectively.

The second trocar 684 can be pivotably coupled to the planetary gear 620b. For example, in some embodiments, a proximal portion of the second trocar 684 can be bent (e.g., have an orthogonal bend) into a shape that provides an axis around which the second trocar 684 can pivot. The second trocar 684 can, for example, be a bent needle. The portion of the trocar forming the pivoting axis can, as shown best in FIG. 9, be coupled to the planetary gear 620b and function similar to the pivot pin 624 for the first trocar 674. Alternatively, the second trocar 684 can be coupled to the planetary gear 620b via a pivot pin, or in any suitable manner.

In some embodiments, as shown best in FIG. 9, the second trocar 684 can be movably coupled to the infusion cannula 680, such as by being inserted into a lumen of the infusion cannula 680. The second trocar 684 and the infusion cannula 680 can be coaxial with each other (e.g., the second trocar 684 can be arranged telescopically within the infusion cannula 680). The second trocar 684 can be in fluid communication with a reservoir (e.g., similar to reservoir 408) such that, when disposed within the lumen of the cannula 680, the cannula 680 is in fluid communication with the reservoir. In some embodiments, the infusion cannula 680 can include a low-friction material on its inner surface (e.g., PTFE) to help ease relative movement of the second trocar 684 and the infusion cannula 680. The second trocar 684 can have a sharpened distal end and made of metal or another suitable rigid material, such that during an insertion process, the second trocar 684 can help puncture skin of a user and enable placement of the infusion cannula 680 in the punctured skin.

Motion of the second trocar 684, and of the infusion cannula 680 that is coaxial with second trocar 684, can be constrained to a linear path at least in part by a cannula carrier 650. For example, the infusion cannula 680 can be coupled to the cannula carrier 650, and the cannula carrier 650 can be slidingly engaged with the housing 610 (e.g., the cannula carrier 650 can include tongue feature(s) that are engaged within a grooved track 616b, or vice versa), such that motion of the cannula carrier 650 is generally limited along one axis aligned with the insertion direction of the second trocar 684 and the infusion cannula 680.

The first and second epicyclic gear arrangements 601a and 601b can have different gear tooth counts for their respective ring gears and their respective planetary gears. For example, as described above, the ring gear 612a and the planetary gear 620a in an example first epicyclic gear arrangement 601a can have a module of about 0.5 (e.g., 0.4792) and a tooth count of 24 and 12, respectively. Additionally, as described above, the ring gear 612b and the planetary gear 620b in an example second epicyclic gear arrangement 601b can have a module of about 0.5 (e.g., 0.4792) and a tooth count of 16 and 8, respectively. In a device having first and second epicyclic gear arrangements with these different respective ring gear tooth counts and different respective planetary gear tooth counts, the difference in the respective tooth counts of the two epicyclic gear arrangements enables the first trocar and second trocar 674, 684 to be inserted to different depths within the user's skin. Furthermore, in a device having such first and second epicyclic gear arrangements that are driven by the same actuator (e.g., torsion spring 630), the first and second epicyclic gear arrangements are configured to drive the first trocar and second trocar 674, 684 different insertion depths simultaneously with a single actuator.

Similar to the sensor carrier 640, the cannula carrier 650 can include one or more latches to selectively limit a position of the cannula carrier 650 relative to the housing 610. For example, the insertion assembly can include a one-way latch that, when engaged, holds the cannula carrier 650 in or near a fully inserted state, thereby holding the infusion cannula 680 in or near the fully inserted state. In some embodiments, the one-way latch includes one or more ratchet features. For example, as shown in FIG. 6B, a backside or other surface of the cannula carrier facing the housing 610 can include one or more flexible arms 652 (e.g., one arm on each side of the cannula carrier 650). Each flexible arm 652 can have a bent free end that is configured to engage lateral latch grooves 614b (latch grooves 614b shown in FIG. 7A). Operation of the flexible arms 652 is similar to that described herein and shown in FIG. 14 with respect to the flexible arms 644a for latching the sensor carrier 640. In other words, the one or more latches are configured to selectively hold the cannula carrier 650 and the infusion cannula 680 in a fully inserted state, but do not restrain the removal of the second trocar 684 from the fully inserted state. Accordingly, the one or more latches enables insertion of the infusion cannula 680 into a user's skin while facilitating subsequent removal of the second trocar 684 from the user's skin. Although a ratcheting latch is shown in the figures, it should be understood that in some embodiments, the epicyclic insertion assembly can additionally or alternatively include other types of latches or one-way securement mechanisms (e.g., cantilevered bars that mate with a feature of the housing 610 and/or device housing 350, adhesive, suitable contact-based fasteners such as hook-and-loop fasteners, etc.).

As shown in FIG. 9, in some embodiments, the cannula carrier 650 can include a housing with an aperture or other suitable cutout that enables the infusion cannula 680 to extend downwardly toward the housing 350 of the infusion device. A cannula septum 689 and/or a funnel 688 can be arranged in the housing of the cannula carrier 650, with respective apertures that are arranged coaxially with the infusion cannula 680. The cannula septum 689 can maintain a hermetic and/or substantially air-tight seal over the cannula 680 while the cannula septum 689 is being pierced by the second trocar 684. With reference to the device orientation shown in FIG. 9, a lower portion of the cannula carrier 650 can be configured to engage (or be received in) a cannula bearing 686 that is arranged on a lower surface of the device housing 350. The cannula bearing 686 can help facilitate axial movement of the infusion cannula outwardly from the device housing 350.

### III. Methods of operation

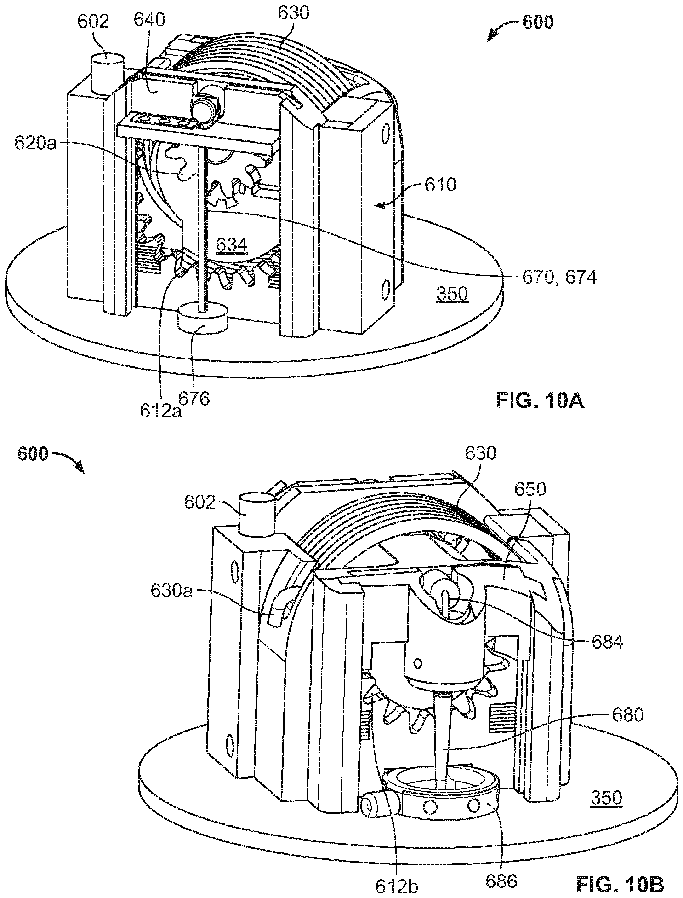

FIGS. 10A-13B illustrate various states of an example epicyclic insertion assembly of an infusion device with an integrated sensor. While the operation of an epicyclic insertion assembly is shown and described with reference to an insertion assembly 600 as described herein, it should be understood that aspects of the epicyclic insertion assembly operation can apply to other variations of the epicyclic insertion assembly.

FIGS. 10A and 10B depict a sensor electrode side and an infusion cannula side, respectively, of an epicyclic insertion assembly 600 in an unfired state. In this state, the epicyclic gear arrangement has not yet moved either the first trocar 674 or the second trocar 684 out of the housing 350 and into a user. In the unfired state, the sensor carrier 640 holds the sensor electrode 670 and the first trocar 674 in a retracted position, with the sensor carrier 640 located at an upper end of its range of linear travel (in the device orientation shown in FIG. 10A). The distal end (e.g., tip) of the first trocar 674 and/or the sensor electrode 670 can be positioned in or adjacent the septum 676, so that the first trocar 674 and/or the sensor electrode 670 can axially travel through the septum 676. Similarly, in the unfired state, the cannula carrier 650 holds the infusion cannula 680 and the second trocar 684 in a retracted position, with the cannula carrier 650 located at an upper end of its range of linear travel (in the device orientation shown in FIG. 10B). The second trocar 684 and/or the infusion cannula 680 are axially aligned with the cannula bearing 686, so that the second trocar 684 and/or the infusion cannula 680 can axially travel through the cannula bearing 686. As shown in FIGS. 10A and 10B, in the unfired state the trigger 602 remains inactivated (cocked), such that the torsion spring 630 is loaded and prepared to actuate rotation of the hub 634a associated with the first epicyclic gear arrangement and rotation of the hub 634b associated with the second epicyclic gear arrangement, once the trigger 602 is activated.

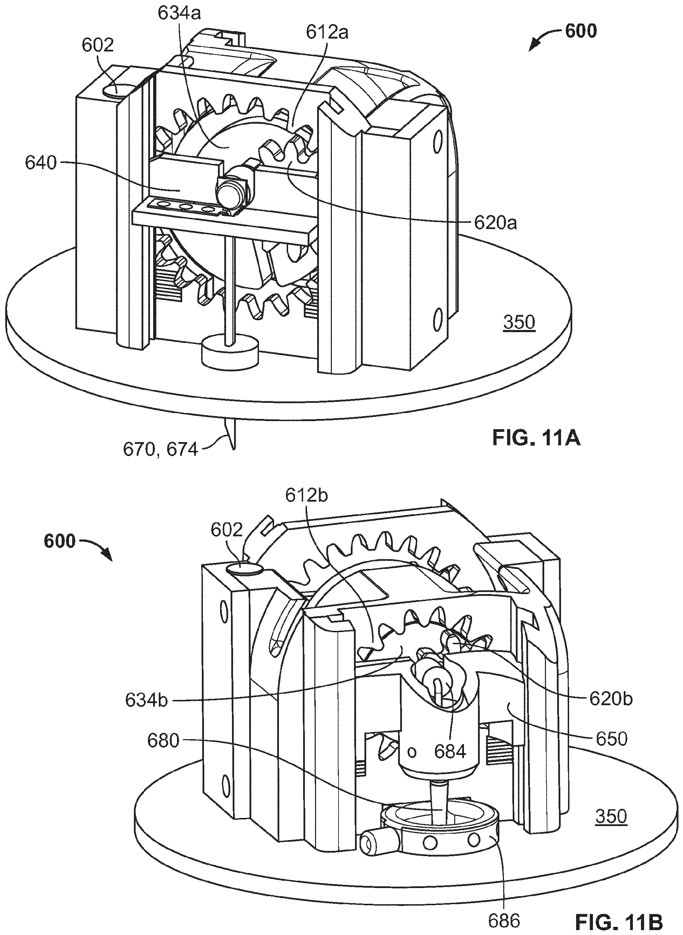

FIGS. 11A and 11B depict a sensor electrode side and an infusion cannula side, respectively, of the epicyclic insertion assembly 600 in an inserted state achieved after the trigger 602 is activated (e.g., the button is depressed) to release the actuator (torsion spring 630, not shown in FIGS. 11A and 11B). The inserted state shown in FIGS. 11A and 11B is after the torsion spring 630 partially unloads (e.g., unwinds) about 60 degrees of rotation (e.g., the change in relative circumferential positions of the ends of the torsion spring 630 is about 60 degrees) relative to the unfired state. This causes the coupled hubs 634a and 634b for the first and second epicyclic gear arrangements to rotate about 60 degrees relative to the unfired state. Such rotation of the hubs 634a and 634b causes linear movement of the sensor carrier 640 and the cannula carrier 650 (downward, in the orientation shown in FIGS. 11A and 11B), thereby driving or advancing the first trocar 674 and the sensor electrode 670 (via the sensor carrier 640) and the second trocar 684 and the infusion cannula 680 (via the cannula carrier 650) at least partially through respective ports in the device housing 350. With 60 degrees of rotational actuation, each of the first and second epicyclic gear arrangements has delivered only a portion of its total available stroke length. For example, in some instances, in an infusion device that is disposed on a user's skin, the epicyclic insertion assembly 600 having the state shown in FIGS. 11A and 11B has only partially inserted the desired insertion segments of the first trocar 674, the sensor electrode 670, the second trocar 684, and the infusion cannula 680 into the user's skin.

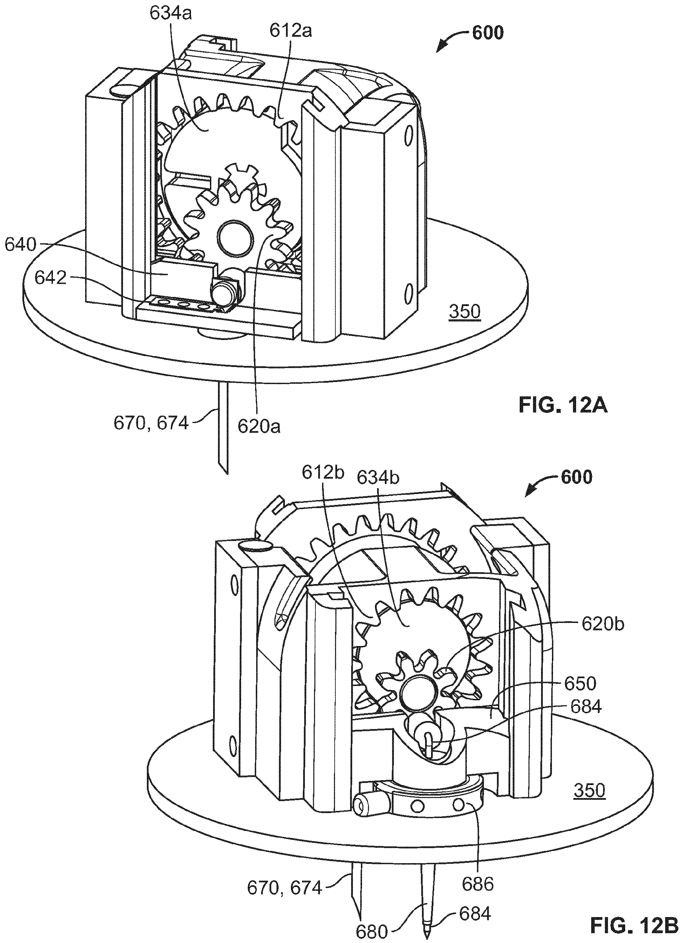

FIGS. 12A and 12B depict the epicyclic insertion assembly 600 in an inserted state achieved after the torsion spring 630 (not shown in FIGS. 12A and 12B) further unloads to about 180 degrees of rotation relative to the unfired state. This causes the coupled hubs 634a and 634b for the first and second epicyclic gear arrangements to rotate about 180 degrees relative to the unfired state, thereby causing linear movement of the sensor carrier 640 and the cannula carrier 650 even further. The linear movement of the sensor carrier 640 and the cannula carrier 650 accordingly drives or advances the first trocar 674 and the sensor electrode 670 (via the sensor carrier 640) and the second trocar 684 and the infusion cannula 680 (via the cannula carrier 650) further through respective ports in the device housing 350, compared to the inserted state shown in FIGS. 11A and 11B. With 180 degrees of rotational actuation, each of the first and second epicyclic gear arrangements has delivered its total available stroke length. For example, in some instances, in an infusion device that is disposed on a user's skin, the epicyclic insertion assembly 600 having the state shown in FIGS. 12A and 12B has fully inserted the desired insertion segments of the first trocar 674, the sensor electrode 670, the second trocar 684, and the infusion cannula 680 into the user's skin. In this fully inserted state, the latches for securing the sensor carrier 640 and the cannula carrier 650 in the fully inserted state are engaged (e.g., flexible arms 644a on the sensor carrier 640 and flexible arms 652 on the cannula carrier engage respective lateral latch grooves on the housing 610). This holds the sensor electrode 670 and the infusion cannula 680 in the fully inserted state, such as in the user's skin.

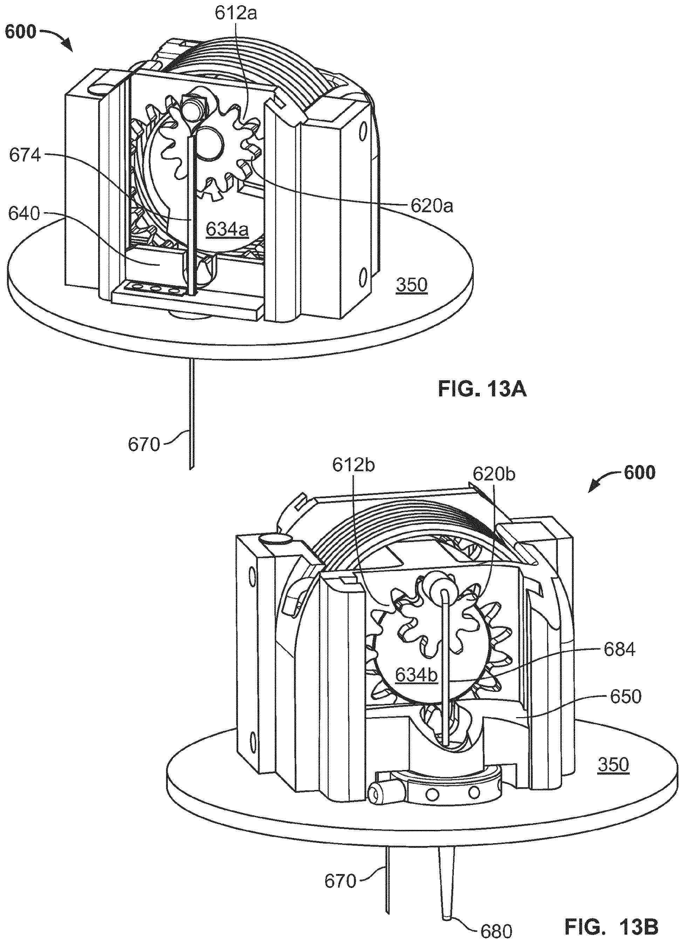

FIGS. 13A and 13B depict the epicyclic insertion assembly 600 in a fully fired state achieved after the torsion spring 630 further unloads to about 360 degrees of rotation relative to the unfired state. The sensor carrier 640 and the cannula carrier 650 have been restrained (as described with reference to FIGS. 12A and 12B) via latches to keep the sensor electrode 670 and the infusion cannula 680 inserted in the user's skin, while the subsequent further actuation of the torsion spring 630 has caused upwards retraction of the first trocar 674 and the second trocar 684.

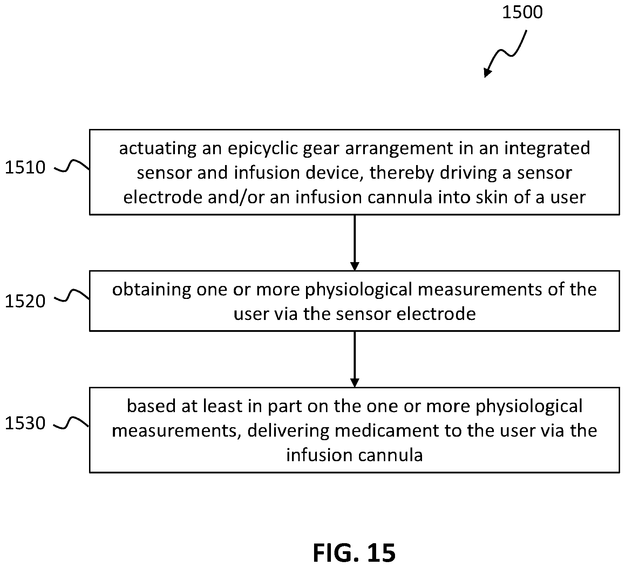

In some embodiments, the operation of the epicyclic insertion assembly as described herein can be performed in connection with treating a user with an infusion device that is arranged over skin of a user. For example, as shown in FIG. 15, a method 1500 can include actuating an epicyclic gear arrangement in an infusion device sensor 1510 with an integrated sensor, where the infusion device includes at least one epicyclic gear arrangement, a sensor electrode, and an infusion cannula fluidically coupled to a reservoir holding a medicament. The epicyclic gear arrangement can, for example, include any of the epicyclic gear arrangements described herein and variations thereof. In some embodiments, the infusion device with the integrated sensor can include multiple epicyclic gear arrangements, as described elsewhere herein. Actuating one or more epicyclic gear arrangements can drive the sensor electrode and/or the infusion cannula into the skin of the user.

The method 1500 can further include obtaining one or more physiological measurements of the user 1520 via the sensor electrode. For example, in some embodiments, the one or more physiological measurements include an analyte measurement (e.g., blood glucose measurement) obtained by the sensor electrode inserted in the user's skin. Furthermore, in some embodiments, the method 1500 can include delivering the medicament to the user via the infusion cannula 1530, based at least in part on the one or more physiological measurements. For example, in some embodiments, the medicament can include insulin that is delivered to the user via the infusion cannula inserted in the user's skin.

Although the devices and methods are described in the context of automatic cannula insertion and patch pumps, it should be appreciated that the techniques are equally applicable to a variety of medical devices (e.g., infusion ports) and to a variety of at least partially implantable devices (e.g., sensors). It should also be noted here that the specification describes structures and methods that are especially well-suited for the subcutaneous delivery of high concentration insulin (i.e., U-200 insulin and above) such as U-500 insulin as well as lower concentration insulin such as U-100 insulin. Nevertheless, it should be appreciated that the present technology is applicable to a wide variety of infusion pumps and medicaments. For example, the present technology is also applicable to medicaments such as, for example, drugs to mask pain, chemotherapy and other cancer related drugs, antibiotics, hormones, GLP-1, glucagon, various other drugs that include large molecules and proteins that may require a high level of delivery accuracy, as well as to relatively high concentration insulin (i.e., U-200 insulin and above) such as U-500 insulin, as well as lower concentration insulin, such as U-100 insulin.

### IV. Conclusion

The descriptions of examples of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

Further disclosed herein is the subject-matter of the following clauses:
1. An infusion device comprising:
   a reservoir configured to hold a medicament;
   a first epicyclic gear arrangement comprising a first output;
   a second epicyclic gear arrangement comprising a second output;
   a first trocar coupled to the first output and movably coupled to a sensor electrode;
   a second trocar coupled to the second output and movably coupled to an infusion cannula, wherein the infusion cannula is fluidically coupled to the reservoir,
   wherein actuation of the first epicyclic gear arrangement drives linear travel of the first trocar and the sensor electrode, and actuation of the second epicyclic gear arrangement drives linear travel of the second trocar and the infusion cannula.
2. The device of clause 1, wherein the first epicyclic gear arrangement comprises a first ring gear and a first planetary gear engaged with the first ring gear, and wherein the second epicyclic gear arrangement comprises a second ring gear and a second planetary gear engaged with the second ring gear.
3. The device of clause 1 or 2, further comprising at least one shaft coupled to one or both of the first and second epicyclic gear arrangements.
4. The device of clause 3, wherein the at least one shaft comprises a first rotatable hub coupled to the first planetary gear and a second rotatable hub coupled to the second planetary gear.
5. The device of clause 4, wherein the first and second rotatable hubs are coupled to each other.
6. The device of clause 2 or of any of the clauses 2 to 5, wherein the first output is the first planetary gear, and the second output is the second planetary gear.
7. The device of clause 1 or of any of the clauses 1 to 6, wherein the first trocar is pivotably coupled to the first output and constrained to move along a first linear path, and wherein the second trocar is pivotably coupled to the second output and constrained to move along a second linear path.
8. The device of clause 1 or of any of the clauses 1 to 7, further comprising an actuator coupled to the first and second epicyclic gear arrangements.
9. The device of clause 8, wherein the actuator comprises a torsion spring.
10. The device of clause 9, wherein a first range of rotation of the torsion spring is configured to drive the first trocar, the sensor electrode, the second trocar, and the infusion cannula in a first direction, and wherein a second range of rotation of the torsion spring is configured to retract the first trocar and the second trocar in a second direction opposite the first direction.
11. The device of clause 8 or of any of the clauses 8 to 10, further comprising a trigger configured to activate the actuator.
12. The device of clause 11, wherein the trigger comprises a manual trigger mechanism.
13. The device of clause 11 or 12, wherein the trigger comprises a remote trigger mechanism.
14. The device of clause 1 or of any of the clauses 1 to 13, further comprising a housing configured to at least partially surround the reservoir, the first epicyclic gear arrangement, the second epicyclic gear arrangement.
15. The device of clause 14, wherein the first trocar, the sensor electrode, the second trocar, and the infusion cannula are configured to linearly travel to extend at least partially outside the housing.
16. The device of clause 14 or 15, further comprising a sensor carrier slidingly engaged with the housing and coupled to the sensor electrode, and a cannula carrier slidingly engaged with the housing and coupled to the infusion cannula.
17. The device of clause 16, further comprising at least one latch configured to selectively limit a position of the sensor carrier, the cannula carrier, or both the sensor and cannula carriers relative to the housing.
18. The device of clause 1 or of any of the clauses 1 to 17, wherein linear travel of the first trocar and the sensor electrode is laterally spaced apart from linear travel of the second trocar and the infusion cannula.
19. The device of clause 1 or of any of the clauses 1 to 18, wherein the sensor electrode is configured to be movably received within a recess of the first trocar.
20. The device of clause 1 or of any of the clauses 1 to 19, wherein the second trocar is configured to extend within a lumen of the infusion cannula.
21. A device comprising:
   a first epicyclic gear arrangement comprising a first ring gear and a first planet gear engaged with the first ring gear;
   a second epicyclic gear arrangement comprising a second ring gear and a second planet gear engaged with the second ring gear;
   a first trocar coupled to the first planet gear and movably coupled to a first medical device; and
   a second trocar coupled to the second planet gear and movably coupled to a second medical device;
   wherein actuation of the first and second epicyclic gear arrangements drives travel of the first and second trocars, respectively.
22. The device of clause 21, further comprising at least one shaft coupled to one or both of the first and second epicyclic gear arrangements.
23. The device of clause 22, wherein the at least one shaft comprises a first rotatable hub coupled to the first planetary gear and a second rotatable hub coupled to the second planetary gear.
24. The device of clause 23, wherein the first and second rotatable hubs are coupled together.
25. The device of clause 23 or 24, wherein the first and second rotatable hubs are splined together.
26. The device of clause 21 or of any of the clauses 21 to 25, wherein actuation of the first epicyclic gear arrangement drives the first trocar in a first linear path and actuation of the second epicyclic gear arrangement drives the second trocar in a second linear path spaced apart from the first linear path.
27. The device of clause 21 or of any of the clauses 21 to 26, wherein the first medical device comprises a sensor.
28. The device of clause 21 or of any of the clauses 21 to 27, wherein the second medical device comprises a cannula.

## Claims

1. An infusion device comprising:
a reservoir configured to hold a medicament;
a first epicyclic gear arrangement comprising a first output;
a second epicyclic gear arrangement comprising a second output;
a first trocar coupled to the first output and movably coupled to a sensor electrode;
a second trocar coupled to the second output and movably coupled to an infusion cannula, wherein the infusion cannula is fluidically coupled to the reservoir,
wherein actuation of the first epicyclic gear arrangement drives linear travel of the first trocar and the sensor electrode, and actuation of the second epicyclic gear arrangement drives linear travel of the second trocar and the infusion cannula.

2. The device of claim 1, wherein the first epicyclic gear arrangement comprises a first ring gear and a first planetary gear engaged with the first ring gear, and wherein the second epicyclic gear arrangement comprises a second ring gear and a second planetary gear engaged with the second ring gear.

3. The device of claim 1 or 2, further comprising at least one shaft coupled to one or both of the first and second epicyclic gear arrangements.

4. The device of claim 3, wherein the at least one shaft comprises a first rotatable hub coupled to the first planetary gear and a second rotatable hub coupled to the second planetary gear, particularly
wherein the first and second rotatable hubs are coupled to each other.

5. The device of one of the claims 2 to 4, wherein the first output is the first planetary gear, and the second output is the second planetary gear.

6. The device of one of the claims 1 to 5, wherein the first trocar is pivotably coupled to the first output and constrained to move along a first linear path, and wherein the second trocar is pivotably coupled to the second output and constrained to move along a second linear path.

7. The device of one of the claims 1 to 6, further comprising an actuator coupled to the first and second epicyclic gear arrangements, particularly
wherein the actuator comprises a torsion spring, particularly
wherein a first range of rotation of the torsion spring is configured to drive the first trocar, the sensor electrode, the second trocar, and the infusion cannula in a first direction, and wherein a second range of rotation of the torsion spring is configured to retract the first trocar and the second trocar in a second direction opposite the first direction.

8. The device of claim 7, further comprising a trigger configured to activate the actuator, particularly
wherein the trigger comprises a manual trigger mechanism, and/or particularly
wherein the trigger comprises a remote trigger mechanism.

9. The device of one of the claims 1 to 8, further comprising a housing configured to at least partially surround the reservoir, the first epicyclic gear arrangement, the second epicyclic gear arrangement.

10. The device of claim 9, wherein the first trocar, the sensor electrode, the second trocar, and the infusion cannula are configured to linearly travel to extend at least partially outside the housing.

11. The device of claim 9 or 10, further comprising a sensor carrier slidingly engaged with the housing and coupled to the sensor electrode, and a cannula carrier slidingly engaged with the housing and coupled to the infusion cannula, particularly
further comprising at least one latch configured to selectively limit a position of the sensor carrier, the cannula carrier, or both the sensor and cannula carriers relative to the housing.

12. The device of one of the claims 1 to 11, wherein linear travel of the first trocar and the sensor electrode is laterally spaced apart from linear travel of the second trocar and the infusion cannula, and/or
wherein the sensor electrode is configured to be movably received within a recess of the first trocar, and/or
wherein the second trocar is configured to extend within a lumen of the infusion cannula.

13. A device comprising:
a first epicyclic gear arrangement comprising a first ring gear and a first planet gear engaged with the first ring gear;
a second epicyclic gear arrangement comprising a second ring gear and a second planet gear engaged with the second ring gear;
a first trocar coupled to the first planet gear and movably coupled to a first medical device; and
a second trocar coupled to the second planet gear and movably coupled to a second medical device;
wherein actuation of the first and second epicyclic gear arrangements drives travel of the first and second trocars, respectively.

14. The device of claim 13, further comprising at least one shaft coupled to one or both of the first and second epicyclic gear arrangements, particularly
wherein the at least one shaft comprises a first rotatable hub coupled to the first planetary gear and a second rotatable hub coupled to the second planetary gear, particularly
wherein the first and second rotatable hubs are coupled together, and/or particularly
wherein the first and second rotatable hubs are splined together.

15. The device of claim 13 or 14, wherein actuation of the first epicyclic gear arrangement drives the first trocar in a first linear path and actuation of the second epicyclic gear arrangement drives the second trocar in a second linear path spaced apart from the first linear path, and/or
wherein the first medical device comprises a sensor, and/or
wherein the second medical device comprises a cannula.
